# EUROPEAN PATENT APPLICATION

(11) **EP 2 130 902 A1**
(43) Date of publication of application: **09.12.2009**
(21) Application number: 08157560.7
(22) Date of filing: 04.06.2008
(51) Int. Cl.: C12M 1/00

(54) **Process for producing microorganisms and apparatus applied therefore**

(71) Applicant: SBAE Industries NV, 9950 Waarschoot (BE)
(72) Inventor: Vanhoutte, Koenraad, 9040 Gent (BE); Vanhoutte, Jan, 8750 Zwevezele (BE)
(74) Representative: Moens, Marnix Karel Christiane

(57) **Abstract**

The present invention is related to a process for producing microorganisms comprising a buffering step between the continuous process step and the batch wise process step.

The present invention is also directed to an apparatus for the production of microorganisms comprising at least one continuous process unit, at least one batch wise process unit and at least one buffer unit provided with an inlet cooperating with an outlet of the continuous process unit and with an outlet cooperating with an inlet of the batch wise process unit.

## Description

### Field of the Invention

The present invention relates to a process for producing microorganisms, particularly for producing algae. The present invention also relates to an apparatus for producing microorganisms.

### Background of the Invention

Microorganisms, which are exceptionally diverse, are found almost everywhere and affect human society in countless ways. Hence, modern microbiology has a great impact on medicine, agriculture, food science, ecology, genetics, biochemistry and many other fields. The basic microbiological knowledge of human society possesses the potential to revolutionize many aspects of human life.

A growing interest in the applications with the enormous variety of microorganism, such as algae, requires an economic and efficient method and accompanying apparatus for producing microorganisms. The present invention is a key resource to underpin these developments.

At present several processes are known for producing algae. US4116778 for example describes a plant for the production of microorganisms, comprising a reactor coupled to a recirculation circuit whereby a nutrient medium with microorganisms suspended therein is continuously circulated in and out the reactor through the recirculation circuit. At defined moments, part of the recirculation circuit is shut off and the broth of microorganisms present therein is collected and replaced by fresh nutrient medium.

An example of a staged continuous-batch process for producing algae is described in US 3763824. The process comprises a plurality of cultivation containers in series arranged in an exponential progression of container volume. In a first process step, algae are cultivated in the smallest container, and once a desired concentration of algae is attained therein, a portion of culture medium is batch wise transferred to the next larger container in series, together with fresh nutrient medium. As such, the transferred culture medium is used as inoculum for the next container. The culture medium removed from the first container is replaced by fresh culture medium. Once the culture medium in the second container has reached a desired concentration of algae, the entire culture medium present therein is used as an inoculum for a next container in the series and the above cycle is repeated, in order to obtain a staged-continuous production process of algae.

A drawback of the above staged continuous-batch processes is that the cultivation step is performed batch wise, while microorganisms grow continuously under appropriate growth conditions. Batch wise cultivation of microorganisms is suboptimal for the quality of the cell content, is inefficient in its yield (cell densities) and is as a whole substantially less economic than continuous cultivation set-up. This approach is furthermore sensitive to degradation of the biomass during waiting periods between harvesting and processing. Not only does this further reduce the economics of the system, but cell damage and rapid product degeneration characterizes micro-algae concentrates that are available on the market today.

Other known processes are exemplified by JP 2005261342 which discloses a process for continuously producing plankton. This known process essentially comprises a continuous cultivation step in a reactor and a continuous harvesting step by filtering the medium broth in a collection tube. Although JP 2005261342 allows efficiently cultivating plankton, the collection and harvesting of the plankton is restricted to a continuous process step such as filtering.
Since most collection and harvesting processing technologies, suitable for processing microorganisms, consist of batch-like processes, the process disclosed in JP 2005261342 seriously limits process design options. Moreover, a single filtration step is often inconvenient for collecting and harvesting biomass as it does not allow sufficient concentration and drying of the collected biomass to prevent rapid degeneration. It is further clear that additional concentration and drying steps will increase production cost en decrease efficiency of the overall production process as storage of the microorganisms between two subsequent concentrations and drying steps will lead to cell lyses, which decreases the quality of the end product.

Simply providing batch wise collection and harvesting process steps at the outlet of the cultivation step does not necessarily provide a more efficient production process. Indeed, batch wise collection and harvesting devices such as for instance; chamber centrifuges are limited by the size of their chamber and the cell density of the centrifuged medium broth, once the chamber is filled up they need to be emptied of biomass, cleaned and reset; membrane filtration is batch-like limited by the saturation of the membranes; freeze dryers are limited by the size of the vacuum chamber etc...

All these collection and harvesting devices operate in batch-mode and as such may form a typical bottle neck in the microorganism production process and cannot be operated in a continuous way. Indeed, there is a lower limit to the size of downstream process devices which implies that sufficient medium broth or biomass must be available in order for the process to function properly and economically. This volumetric limitation in conjunction with the fact that there is an inherent upper limit to the production capacity of microorganisms per liter medium automatically defines the minimal size of an economically healthy factory for mass production of microorganisms. The resulting size implies virtually prohibitive capital investment costs for the start up of mass production of microorganisms. This problem has repeatedly been identified in academic pilot plants as the prohibitive constraint for mass production of microorganisms (e.g. Molina Grima et al. Richmond 2005.). The problem prevents the larger population of having commercial access to the beneficial products derived from micro-algae.

The present invention addresses a process and apparatus applied for producing microorganisms which sustains the cell quality and cell density while a volume sufficient for downstream processing is collected providing a solution to one or more of the above mentioned drawbacks.

The present invention also addresses a controllable and structured circulation pattern independent of the level of filling of the system.

The present invention further addresses a controlled flashing light effect to the cultured microorganisms.

### Summary of the Invention

The present invention is directed to a process for producing microorganisms comprising at least one continuous process step, at least one batch wise process step and at least one buffering step between said continuous and batch wise process step.

Further the present invention is directed to an apparatus for producing microorganisms, the apparatus comprising at least one continuous process unit, at least one batch wise process unit and at least one buffer unit provided with an inlet cooperating with an outlet of the continuous process unit and with an outlet cooperating with an inlet of the batch wise process unit.

In accordance with another embodiment, the present invention is also directed to a bioreactor for production of microorganisms, comprising a housing, means for circulating a suspension of microorganisms therein, characterized by said means comprising elements stacked and/or packed in the housing thereby creating at least one central channel.

### Description of the Invention

The present invention is directed to a process for producing microorganisms comprising at least one continuous process step, at least one batch wise process step and at least one buffering step between said continuous and batch wise process step.

It has now been surprisingly found that the incorporation of a buffering step between the continuous production step and the batch wise processing step allows combining a continuous cultivation step with a batch wise collection and harvesting step.

An advantage of the process according to the present invention is that microorganisms can be stored while sustaining cell quality and cell vitality until a volume, sufficient for economic downstream processing, is reached.

Preferably, the buffering step comprises circulating a suspension of said microorganisms provided by the continuous process step in a buffer unit. This circulation of the suspension is preferably controllable by elements stacked and/or packed in the buffer unit and even more preferably an air flow is generated through the suspension of microorganisms and/or packaging and/or stacking in the buffer unit. Circulating the suspension of microorganisms offers the advantage that the circulation is beneficial for sustaining cell quality and cell vitality in the buffer unit until a volume, sufficient for economic batch wise downstream processing, is reached.

A preferred process according to the invention comprises exposing the microorganisms in the buffer unit to light. Combining this preferred process with controlled circulation of the suspension of microorganisms in the buffer unit has surprisingly been found to be advantageous for sustaining their cell quality and cell vitality. It is believed that this advantage is due to the fact that combining both exposing the microorganisms to light and circulating them in a controlled manner creates what is called a 'flashing light effect' that is a known photosynthetic growth mechanism for microorganisms.

### Brief description of the Drawings

- Figure 1: schematically represents an apparatus according the present invention for producing microorganisms;
- Figure 2: schematically represent a bioreactor applicable in an apparatus according the invention;
- Figure 3: schematically represents a buffer unit applicable in an apparatus according the invention;
- Figure 4: schematically represents a conical element comprising a top opening and a bottom opening;
- Figure 5: schematically represents gradual filling of the buffer unit of figure 3;
- Figure 6: schematically represents another embodiment of the buffer unit of figure 3;
- Figure 7: schematically represents an alternative embodiment of the buffer unit in the process. A first buffer unit is placed next to but separate of the second buffer unit.
- Figure 8: schematically represents another embodiment of the buffer unit in the process. A first buffer unit is placed inside and central in the second buffer unit.

### Definitions

In the context of the present invention, 'production' should be understood as growing microorganisms in a culture broth and in a later stage subjecting the culture broth to a further downstream processing such as, for example, collecting the microorganisms from the culture broth or stimulating the microorganisms to synthesize specific components (e.g. carotenoids).
'Continuous' is to be understood as non-stop. Continuous production is the continuous supply of nutritionally complete medium to an inoculum in a bioreactor with subsequent non-stop generation and collection of microorganisms. In contrary, 'batch wise' is to be understood as step-wise. A batch wise production process is a discontinued process wherein microorganisms are grown and collected dependent on the size of the bioreactor and/or the downstream processing device. At the time of collecting the microorganisms from the bioreactor, the supply of nutrients to the culture medium is stopped. Usually, the size of the downstream process devices determines the level of production of microorganisms and hence, is the bottle neck for economic scale production of microorganisms.

'Collecting' is to be understood as gathering the culture broth with microorganisms suspended therein out of a bioreactor, whilst 'harvesting' indicates concentration (e.g. drying, filtering) of the microorganisms collected from the bioreactor.

The 'buffering step' should be understood as an intermediate step between the step of growing microorganisms in a bioreactor and the step of collecting the microorganisms for downstream processing. Said buffering step is useful to sustain the cell quality and cell density of the microorganisms while a volume sufficient for downstream processing is obtained.

According to the invention microorganisms are produced. For the purpose of this invention preferred micro-organisms are those organisms, both single-celled and multi-cellular organisms, as well as non functional colonies of single celled organisms.

### Description of a Preferred Embodiment

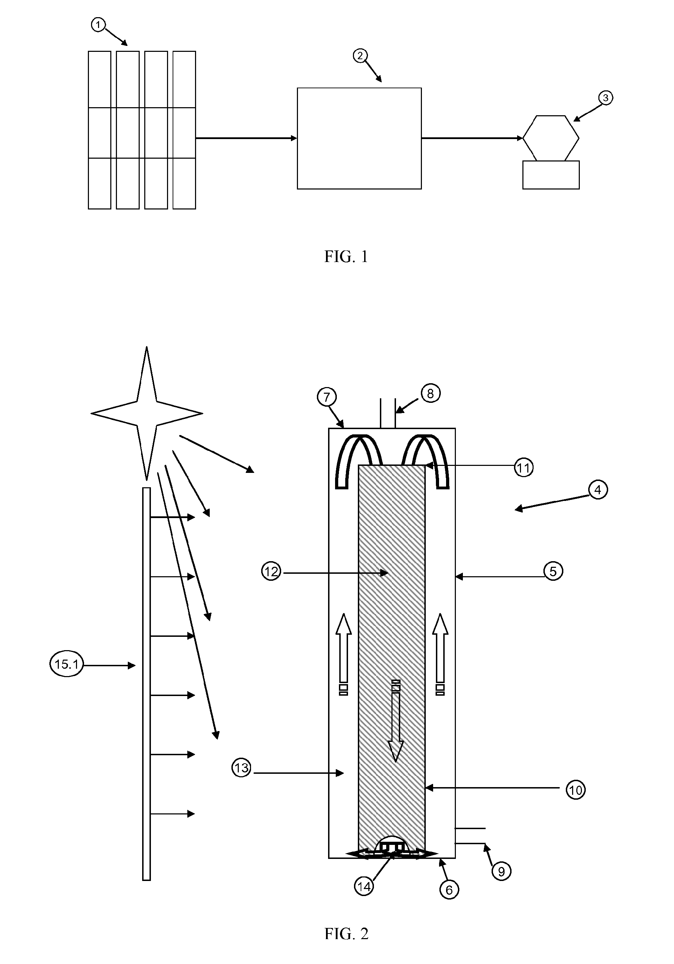

Referring to figure 1, and in accordance with the present invention, an apparatus for producing microorganisms is presented, the apparatus comprising at least one continuous process unit 1, at least one buffer unit 2 and at least one batch wise process unit 3.

In the present embodiment the continuous process unit comprises a battery of bioreactors 4 arranged in parallel. One such bioreactor 4 is illustrated more in detail in figure 2 and is of a double cylinder photo-bioreactor type. This photo-bioreactor 4 comprises an outer cylinder 5 that is vertically positioned on a base 6 and is covered at its open end by a top disc 7. The base 6, the outer cylinder 5 and the top disc 7 are fixed to each other in a sealing manner and define the reactor space.

In the top disc 7 an opening is provided communicating with an inlet 8 of the bioreactor 4, while in a lower part of the outer cylinder 5 an outlet opening is provided that is in communication with an outflow tube 9.

Vertically inside the reactor space is provided a central cylinder 10, fixed to the base plate 6. This central cylinder 10 is shorter than the outer cylinder 5 and has an open end at its top 11. The central cylinder 10 divides the reactor space in an inner channel 12 within the central cylinder 10 and an outer channel 13 in between the outer and inner cylinders 5 and 10. In the lower end of the inner cylinder 10 are provided some discrete insertions 14 defining flow through openings between the inner channel 12 and the outer channel 13.

Preferably a light source 15.1 is provided outside the bioreactor 4 and directed to the outer cylinder 5. Even more preferred are means for circulating a suspension of microorganisms comprising means (not shown in the figures) for generating an air flow in the bioreactor 4. Preferably, spargers may be placed in the central cylinder 10. Alternatively air channels can be provided in the central cylinder or in the outer cylinder, said air channels being in fluid communication with an air inlet and with openings extending in to the central channel or outer channel for blowing air into either or both central and outer channels.

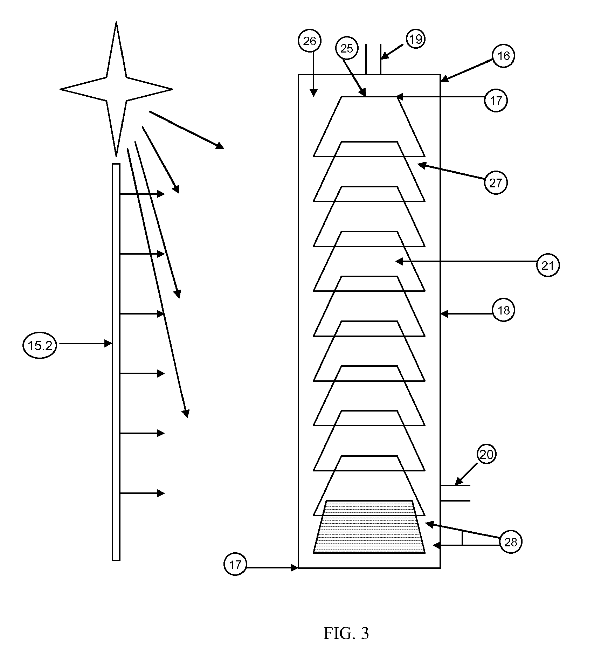

Figure 3 schematically represents a buffer unit 2 comprising a reservoir 16 having a base 17 and an upright, preferably cylindrical, wall 18. The reservoir 16 further comprises an inlet 19 that is connected to the outflow tube 9 of the bioreactor 4 and an outlet 20.

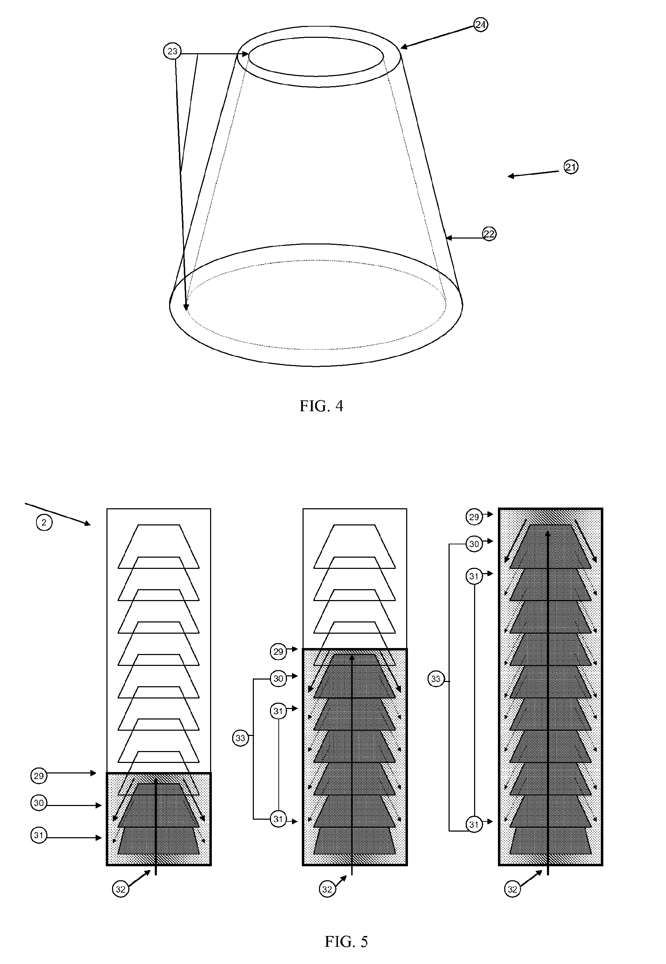

According to a preferred embodiment of the present invention, the buffer unit comprises means for circulating a suspension of microorganisms present therein. In this case and with reference to figure 3, said means comprise elements 21 stacked or packed in the reservoir 16. These elements 21, an example of which is represented in figure 4, preferably have a conical side wall 22 defining a through channel 23. In a stacked configuration, several of said elements 21 are positioned in an upright pile one on top of the other, with their narrow end 24 pointing upwards. As such, the slopes of the stacked elements 21 are aligned with the water flow in a cocurrent way. As such the slits through channels 23 of the different elements are aligned and create a central channel 25 within the reservoir 16. A peripheral channel 26 can than be defined as limited by the side walls 22 of the stacked elements 21 and the cylindrical wall 18 of the reservoir 16.

The stacked elements 21 are preferably spaced apart over a distance of at least some millimeters, such that their side walls 22 do not contact each other and lateral openings 27 are defined between two adjacent elements 21, thereby creating a communication between the central channel 25 and the peripheral channel 26. The spacing apart can be achieved by providing spacing elements on the side walls 22 of the elements 21 or in any other convenient manner well known to a person skilled in the art. The lowermost of the stacked elements, id est the element 21 positioned closest to the base 17 of the reservoir 16 preferably comprises a number of slits 28.

In a preferred embodiment in accordance with the present invention, the means for circulating a suspension of microorganisms comprise a means (not shown in the figures) for generating an air flow in the buffer unit 2. Preferably, spargers may be placed in the central channel 25 of the reservoir 16. Alternatively, mechanical mixers or rotors may be employed.

Preferably a light source 15.2 is provided outside the buffer unit 2 and may be directed to the cylindrical wall 18 and/or perpendicular to the top of the reservoir 16. It is clear that in this case the wall 18 of the reservoir 16 has to be translucent. Sprinklers (not shown in the figures) may also be positioned outside the reservoir 16, whereby the heads of the sprinklers are directed so that the elements 21 can be rinsed.

The outlet 20 of the buffer unit 2 is in communication with an inlet of the batch wise process unit 3. Said batch wise process unit may for example comprise a plate centrifuge, a freeze-dryer, etc. Such batch wise processes are well known for a person skilled in the art and will not be described in further detail.

Starting the process for producing microorganisms with the apparatus as described above, a first step is the continuous process step of cultivating microorganisms. Microbial cells are produced in the photo-bioreactor 4. Preferably, the continuous process unit 1 is configured such that there is continuous dilution of the cell culture by continuously supplying fresh medium containing nutrients to the photo-bioreactor through the inlet 8.

As nutritionally complete medium is continuously supplied to the photo-bioreactor 4, the culture medium rises gradually. To optimize the growth conditions for the microorganisms, the culture medium is mixed through spargers provided in the outer channel 13. The spargers generate an air flow which causes an air lift system, as illustrated in figure 2.

This air lift system generates an upward flow of the suspended cells in the outer channel 13. When the culture medium reaches the top 11 of the central cylinder 10, the medium flows downward in the inner channel 12. At the bottom of the inner channel 12, the cells are dispersed in the insertions 14 and subsequently pushed back upwards in the outer channel 13 by the continuous air lift system. As such, the air lift system allows for circulation patterns to improve the flashing light effect and to homogenize the suspension.

At the time the bioreactor 4 reaches its full capacity, i.e. at a desired volume and/or cell density, the medium broth is continuously transferred via the outlet 9 of the bioreactor 4 and continuously captured in the buffer unit 2 via the cooperating inlet 19. The volume of nutrient medium supply in the photo-bioreactor 4 equals the volume of medium broth transferred to the buffer unit 2.

This buffer unit 2 is especially designed to hold the transferred medium broth and sustain cell density, cell vitality and cell content. By comprising this buffering step between the continuous process step and the batch wise process step, a volume, sufficient for economic downstream processing, can be reached. Temporarily storing the medium broth in the buffer unit 2 allows the combination of a continuous cultivation step with a batch wise collection and subsequent harvesting step.

Sustaining the cell quality and cell vitality is enhanced by circulating the suspension in the buffer unit 2. Through continuous transfer of the suspension of microorganisms from the bioreactor 4 to the buffer unit 2, the cell culture in the buffer unit 2 gradually rises. The spargers in the central channel 25 of the reservoir 16 diffuse air bubbles generating a central air lift system creating an upward flow of medium both in the central channel 25, shown in figure 5. The central air stream will vigorously mix the liquid in the buffer unit 2.

At the time the suspension in the central channel 25 reaches the lowermost slit of the lowermost element 21, circulation may start. The suspension flows upward in the central channel 25, through the slit 28 towards the peripheral channel 26, where it flows downward again to be reentered in the central channel through openings provided between the lowermost element and the base 17 to close the circulation loop. With increasing suspension level corresponding, circulation loops will occur for the subsequent slits 28 in the lowermost element 21. The multiple lateral slits 28 in the lowermost element 21 will create sufficient turbulence to ensure sufficient intermediate circulation until the lowermost element 21 overflows, hereafter the lateral openings 27 between the subsequent elements 21 will take over the top of the slits. As the level of the suspension in the buffer unit 2 gradually rises, circulation patterns are created, independent of the level of filling of the buffer unit 2.

By stacking and/or packing the elements 21, the circulation is improved. When the top level of the liquid medium 29 reaches the narrow end 24 of an element 21, the medium is spilled over that end causing a top overflow, as illustrated in figure 5. Said top overflow causes a dominant water-stream 30 which dominates the overall circulation pattern. Liquid spilled over the top element 21 dominates the streaming of the liquid that flows through the lateral openings 27, defined as subdominant water-streams 31. Once the microorganisms reach the base 17 of the peripheral channel 26, they are pushed again in the central channel 25. Hence, in the peripheral channel 26, a downward flow 33 is created, while in the central channel 25, an upward flow 32 is created. While the culture volume rises, this process is repeated until the last element 21 is included in the circulation pattern. In this way the apparatus allows for a continuous and controllable circulation to be maintained independent of the level of filling.

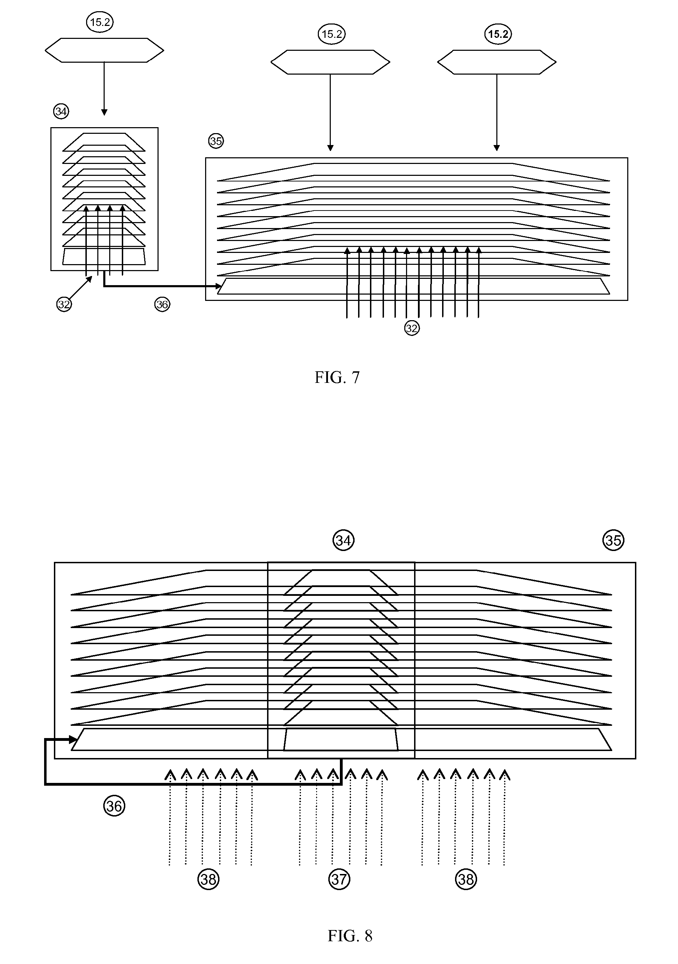

By providing the buffer unit 2 with the elements 21 having conical side walls 22, different advantages can be achieved. Firstly, as illustrated in figure 7, the downward water flow in the peripheral channel 26 is continuously forced to the cylindrical wall 18 of the reservoir 16 because the water flow encounters the sloping sides of the elements 21. Consequently organisms in this water-stream are also continuously pushed towards the cylindrical wall 18, encountering the light source 15.2. These organisms are thus continuously pushed into a photo-active zone. This type of circulation pattern is beneficial for the phototropic growth of autotrophic organisms. Turbulence patterns generated by this water current re-circulate the organisms in the peripheral channel 26 in a very efficient way, and as such promote what is called the flashing light effect. The present invention is based at least partially on microorganisms requiring sustained optimal growth conditions before they are harvested or removed from the spent medium, particularly during buffering of the microorganisms. As photosynthetic microorganisms require a controlled light regime for the conversion of light energy into chemical energy, said light regime also known as the flashing light effect, this effect can be provided by exposing the buffer unit 2 to light. The light source 15.2 guarantees that microorganisms in the peripheral channel 26 are exposed in a photo-active zone, thus a day regime, while microorganisms in the central channel 25, are not exposed to light, and hence are in the night regime. This effect is beneficial for the growth of said microorganisms.

Secondly, the outer downward water flow 33 and the central upward flow 32 interact inside each lateral opening 27. The inherent dynamics of the central air-stream and the outer turbulent water-stream 33 will result in a constant shifting of possibly occurring still points which in its turn will prevent loading of settled material at any given location. Also, due to the sloping sides of the elements 21 any settling material will slip downward towards the peripheral channel 26, where it will be pulled into the downward water stream 33. In addition, the cone-like elements 21 are of such a shape as to prevent air bubbles from the central channel 25 to be drawn through the lateral openings 27 into the cylindrical wall 18 and as such prevent that these bubbles interfere with the downward water flow 33.

It is noteworthy that during the interval where the medium level rises inside an element 21, the combination between turbulence, air bubble dynamics and column pressure will ensure that all organisms will circulate through the peripheral channel 26. The difference in height of the surface levels between the central channel 25 and the peripheral channel 26 illustrates the draft forces that move all organisms to the peripheral channel 26 downward and from there again in the central channel 25. This controlled and structured circulation allows providing the flashing light effect.

Once the buffer unit 2 has reached a certain volume, the full content of the buffer unit 2 may be transferred via the outlet 20 to an inlet of the batch wise downstream process unit 3 where it is processed as one single batch. The volume of the buffer unit 2 preferably stands in relation to the capacity or volume of the first occurring process downstream.

An example of a first downstream process 3 could typically be separation of micro-algae from liquid medium by plate centrifugation. Here, the suspended biomass is separated from the medium liquid. Other subsequent processes can be added at will. The invention as presented here allows for the downstream processes to be performed in a batch wise nature with existing downstream processing technologies: membrane technology, centrifugation techniques, drying techniques, extraction processes, etc.

A possible variation of the embodiment according to the present invention is the buffering process comprising an intermediate stage for secondary treatment of the microbial biomass. It is known to those skilled in the art that a number of treatments can be performed in order for the microbial biomass to change its cell content, cell physiology and/or overall condition, etc.

Research into the mechanisms involved in the sensing and responses of microorganisms to changes in their environments is currently very active in a large number of laboratories worldwide. An increasingly wide range of prokaryotic and eukaryotic species are being studied with regard to their sensing of diverse chemical and physical stimuli, including but not limited to nutrients, toxins, intercellular signaling molecules, redox indicators, light, pressure, magnetic fields, temperature, and surface contact, leading to adaptive responses affecting motile behavior, gene expression and/or development.

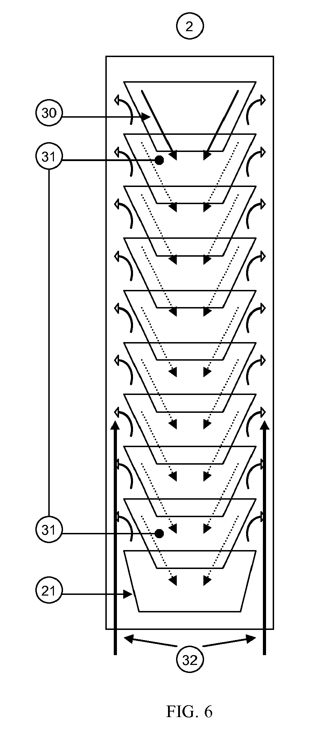

In an alternative embodiment according to the present invention, the cone-like elements 21 are stacked and/or packed with therein the narrow ends 24 pointed downwards, as illustrated in figure 6.

This configuration requires a circumlateral or annular air supply, such that an upward water flow 32 inserted in the peripheral channel 26, encountering the tilted, result of packing and/or stacking the elements 21, conical side walls 22 of the elements 21. As a result the water flow is pushed outward towards the photo-active zone, resulting in a controlled flashing light effect.

In another embodiment of the present invention, it could be envisaged to have some sort of special shaped or formed objects attached to the bottom inside the central channel 25 and/or the peripheral channel 26. This would be beneficial for the water level to initially rise faster and to reach the top opening of the lowermost element 21 sooner. This could be either considered as an alternative to the multiple lateral slits 28 for the lowermost element 21, but can equally be considered as to work in conjunction with the multiple lateral slits 28.

Typically but not shown in the figure, a light source may be provided outside the buffer unit and may be directed to the wall of the cylinder and/or perpendicular to the top of the cylinder. Any type of light source appropriate for exposing the microorganisms in the buffer unit to light, such as an incandescent light bulb or a LED light source, can be used in the present invention. The light source may be encapsulated.

The modular central channel 25, built up by the different elements 21 packed and/or stacked, could be incorporated in any buffer unit, irrespective to the ground plan thereof.

Depending on the preferred first downstream process that is selected, it can be envisaged that more than one buffering step may be inserted between continuous process step 1 and the batch wise process step 3, as illustrated in figure 7.

The volume of the first buffer unit 34 preferably stands in relation to the specific biology of the cell culture. More specifically and typically, this volume is between 10-50% of the photo-bioreactor volume. The second buffer unit 35 is designed to hold a larger volume of transferred medium broth and equally sustain cell density, cell vitality and cell content indifferent of the collected volume of medium broth. Further additional buffer units can be provided both in series and in parallel. The volume of said second buffer unit 35 is typically 2 to 20 times of the first buffer unit 34.

In case more than one buffer unit is part of the plant, several embodiments are possible. Describing a plant consisting of 2 buffer units, differentiation is made between two main forms: the first form consists of the first buffer unit 34 and the second buffer unit 35 to be clearly separate devices, connected by pipings, tubings or the like 36, as shown in figure 7. The second form, illustrated by figure 8 consists of a mutual integration of the first buffer unit 34 and second buffer unit 35 in such a way that they are part of one single device. In this embodiment, the excess medium broth is transferred from the first buffer unit 34 to the second buffer unit 35 by pipings, tubings or the like 36. The buffer unit 34 comprises an initial air supply 37, initially generating an air lift system in said buffer unit 34 until the medium broth is transferred. Subsequently, the air supply 37 is also functional for the second buffer unit 35, which contains the transferred medium broth. The second buffer unit 35 comprises an additional air supply 38, proper to said buffer unit only, in addition to the first buffer unit air supply 37.

By including at least one buffering step between the continuous process step and the batch wise process step, and further including at least one buffer unit in the apparatus for microorganism production, microbial cells can be continuously stored. While waiting for subsequent downstream processing, the microorganisms sustain their cell densities and cell content while rapid degeneration of the cells and content is avoided. The microorganisms may remain in the buffer unit until a volume sufficient for a downstream process step is reached. Hence, downstream processing of the microbial cells is no longer a bottle neck in economic and efficient production of microorganisms.

According to another embodiment, the present invention is also directed to the element 21, illustrated in figure 4, for use in the apparatus as described above.

According to a further embodiment, the present invention is directed to a bioreactor for the production of microorganisms, comprising a housing, means for circulating a suspension of microorganisms therein, **characterized in that** said means comprising elements stacked and/or packed in the housing thereby creating at least one central channel.

Although this invention is conceived in a downstream setting of buffer units, it is clear that this invention can be successfully employed in a bioreactor that employs structured circulation patterns, at any phase of the production of microorganisms.

In the present embodiment the bioreactor is of a double cylinder photo-bioreactor type. This photo-bioreactor comprises an outer cylinder that is vertically positioned on a base and is covered at its open end by a top disc. The base, the outer mantle and the top disc are fixed to each other in a sealing manner and define the reactor space.

In the top disc an opening is provided communicating with an inlet of the bioreactor, while in a lower part of the outer cylinder an outlet opening is provided that is in communication with an outflow tube.

The bioreactor preferably comprises means for circulating a suspension of microorganisms present therein. According to the present invention, said means comprise elements stacked or packed in the reactor space. These elements preferably have a conical side wall defining a through channel. In a stacked configuration, several of said elements are positioned in an upright pile one on top of the other, with their narrow end pointing upwards or downwards. As such the through channels of the different elements are aligned and create a central channel within the reactor space. A peripheral channel can than be defined as limited by the side walls of the stacked elements and the wall of the outer cylinder.

The stacked elements are preferably spaced apart over a distance of at least some millimeters, such that their side walls do not contact each other and lateral openings are defined between two adjacent elements, thereby creating a communication between the central channel and the peripheral channel. The spacing apart can be achieved by providing spacing elements on the side walls of the elements or in any other convenient manner well known to a person skilled in the art. The lowermost of the stacked elements, id est the element positioned closest to the base of the reactor space preferably comprises a number of slits.

In a preferred embodiment in accordance with the present invention, the means for circulating a suspension of microorganisms comprise a means (not shown in the figures) for generating an air flow in the buffer unit. Preferably, spargers may be placed in the central channel of the reactor space. Alternatively, mechanical mixers or rotors may be employed. This allows for circulation patterns to improve the flashing light effect and to homogenize the suspension.

Typically a light source is provided outside the bioreactor and may be directed to the wall of the cylinder and/or perpendicular to the top of the cylinder.

An airlift bioreactor that incorporates a circulation system by means of a double cylinder system is normally only operational if the reactor is filled up higher than the top of the central channel. If the water level is lower the system locks and as a result of stagnation the produced organisms degrade rapidly. This phenomenon frequently occurs in traditional systems as the flow of air generated by the airlift results in substantial evaporation. This often inadvertently results in waterlocking, which in turn stops the circulation and is substantially detrimental to the cell culture.

As the liquid medium rises in the bioreactor, water is moved upward in the central channel and when the top of the element is reached, the water moves downward in over the sides of the element.
By stacking and/or packing the elements, the circulation is improved. Not only is the liquid spilled over the top, but also the liquid flows through the lateral openings downwards. This creates a controllable circulation of the suspension of microorganisms in the bioreactor, independent of the level of filling.

The elements are preferably conical. This to ensure the water-stream to be pushed continuously to the outer channel of the bioreactor, to avoid settling of the material at any given location and prevent air bubbles from the central cylinder to be drawn through the lateral openings into the outer channel.

The bottom element in a series of elements packed and/or stacked may also be provided with many lateral slits already integrated in the sides. This may improve the circulation during the initial phase of filling up the bioreactor. This can be necessary because when the top opening of the bottom element is not yet reached, no circulation pattern occurs. Multiple lateral slits in the bottom element will create sufficient turbulence to ensure sufficient intermediate circulation until the lowermost element overflows.

With these embodiments of a buffer unit and a bioreactor include all other forms and shapes that are not circular in ground plan and cylindrical in body and have a central channel system. For instance this could encompass an inverted pyramidal shape with a central segmented column consisting of small pyramids. This could also encompass a large rectangular shape. The advantage of this varying embodiment is the adaptation according to the needs and space available to produce microorganisms.

Having described the process and accompanying apparatus for the production of microorganisms, it is believed that other processes and/or apparatus comprising a buffering step, respectively a buffer unit, will be suggested to those skilled in the art in view of the description set forth above. It is therefore to be understood that all such processes and/or apparatus are believed to fall within the scope of the invention as defined in the appended claims.

A person skilled in the art will also understand that the embodiments described above are merely illustrative in accordance with the present invention and not limiting the intended scope of the invention. Other embodiments and applications may also be considered.

## Claims

1. A process for producing microorganisms comprising
a) at least one continuous process step
b) at least one batch wise process step
c) at least one buffering step between said continuous and batch wise process steps.

2. The process according to claim 1, the buffering step comprising circulating a suspension of said microorganisms provided by the continuous process step a) in a buffer unit.

3. The process according to claim 2, wherein the circulation of the suspension is controllable by elements stacked and/or packed in the buffer unit.

4. The process according to claim 2 or 3, comprising generating an air flow through the suspension of microorganisms and/or packaging and/or stacking in the buffer unit.

5. The process according to any of claims 2 to 4, comprising exposing the microorganisms in the buffer unit to light.

6. A buffering process, as identified in claims 1 to 5, said buffering process comprising circulating a suspension of microorganisms provided by the continuous process step a) in a buffer unit.

7. A buffering process, as identified in claims 1 to 6, said buffering process comprising an intermediate stage for secondary treatment of the microbial biomass.

8. An apparatus for producing microorganisms, the apparatus comprising
a) at least one continuous process unit
b) at least one batch wise process unit
c) at least one buffer unit provided with an inlet cooperating with an outlet of the continuous process unit and with an outlet cooperating with an inlet of the batch wise process unit.

9. The apparatus according to claim 8, wherein the buffer unit comprises means for circulating a suspension of microorganisms therein.

10. The apparatus according to claim 9, wherein said means for circulating the suspension of microorganisms comprise a means for generating an air flow in the buffer unit.

11. The apparatus according to any of claims 8 to 10, wherein elements are stacked and/or packed in the buffer unit creating at least one central channel.

12. The apparatus according to claim 11, wherein the elements are conical.

13. The apparatus according to claim 11, wherein a series of stacked and/or packed elements provide lateral openings communicating with the central channel.

14. A buffer unit as identified in any of claims 11 to 13.

15. An element for use in an apparatus as identified in claims 8 to 13.

16. A bioreactor for production of microorganisms, comprising:
a) a housing;
b) means for circulating a suspension of microorganisms therein, **characterized by** said means comprising elements stacked and/or packed in the housing thereby creating at least one central channel.
